# EUROPEAN PATENT APPLICATION

(11) **EP 4 628 113 A1**
(43) Date of publication of application: **08.10.2025**
(21) Application number: 24900367.4
(22) Date of filing: 07.11.2024
(51) Int. Cl.: A61L 33/06, C08F 220/16, C08F 220/28, C08F 290/06, C08J 7/00, C08J 7/04

(54) **METHOD FOR PRODUCING ANTI?THROMBOGENIC MATERIAL**

(30) Priority: 05.12.2023 JP 2023205501
(71) Applicant: Toyobo Co., Ltd., Kita-ku Osaka-shi, Osaka 530-0001 (JP)
(72) Inventor: KOZAWA, Ayami, Yokkaichi-shi, Mie 510-0106 (JP); KAJII, Fumihiko, Yokkaichi-shi, Mie 510-0106 (JP)
(74) Representative: Vossius & Partner Patentanwälte Rechtsanwälte mbB
(86) International application number: PCT/JP2024/039622
(87) International publication number: WO 2025/121072

(57) **Abstract**

The objective of the present invention under such circumstances is to provide a method for producing an antithrombogenic material having less white turbidity, a method for producing a medical device coated with the medical device produced by the method, and a medical device having less white turbidity. The method for producing an antithrombogenic material according to the present invention is characterized in comprising the step of copolymerizing the specific alkyl (meth)acrylate, silicone (meth)acrylate and alkoxypolyethylene glycol (meth)acrylate, wherein the weight average molecular weight of the silicone (meth)acrylate is 1450 or less.

## Description

### TECHNICAL FIELD

The present invention relates to a method for producing an antithrombogenic material having less white turbidity, a method for producing a medical device coated with the antithrombogenic material produced by the method, and an antithrombogenic material having less white turbidity.

### BACKGROUND ART

In recent years, a medical device utilizing various polymeric materials has been developed and expected to be used as a blood filter, artificial kidney, a plasma separator, a catheter, artificial lung, artificial blood vessel, an adhesion prevention membrane, artificial skin or the like. Such a medical device is required to have biocompatibility in such cases, since a synthetic material, which is foreign to a living body, is used with contacting with a body tissue and blood.

When a medical device containing a material to be contacted with blood is used, 3 items of (a) suppression of a blood coagulation system, (b) suppression of adhesion and activation of blood platelet, and (c) suppression of complement activation are important as biocompatibility. In particular, the suppression of activation of blood platelet and complement described as (b) and (c) is important in the case where a device for extracorporeal circulation, such as artificial kidney and a plasma separator, that is contacted with blood for a relatively short time is used, since an anticoagulant such as heparin and sodium citrate is generally used at the same time.

The applicant of this application developed an antithrombogenic material containing a (meth)acrylate copolymer that is produced by polymerizing the specific (meth)acrylate monomer and that is water-insoluble viscous liquid at room temperature (Patent document 1).

In addition, the applicant of this application developed an antithrombogenic material containing a (meth)acrylate copolymer that is contacted with blood for a relatively long time. The copolymer is produced by polymerizing the specific (meth)acrylate monomer, has the specific weight average molecular weight, and is water-insoluble viscous liquid at room temperature (Patent document 2). Further, the applicant developed a catheter excellent in blood compatibility and biocompatibility (Patent document 3). Even when the catheter is indwelled in the body for a long time, the physical property and scientific property of the catheter do not become poor. At least the part of the catheter to be contacted with body fluid is covered with the antithrombogenic material composed of the specific (meth)acrylate copolymer.

In addition, Patent document 4 discloses an antithrombogenic medical coating material containing the copolymer having the specific segment. Patent document 5 discloses a phosphorylcholine analog group-containing polymer that has the specific structural unit and weight average molecular weight and that is excellent in biocompatibility.

### PRIOR ART DOCUMENT

### PATENT DOCUMENT

Patent document 1: JP 2008-289864 A
Patent document 2: WO 2022/210759
Patent document 3: JP 2009-261437 A
Patent document 4: WO 2019/142710
Patent document 5: JP 2002-356519 A

### SUMMARY OF THE INVENTION

### PROBLEMS TO BE SOLVED BY THE INVENTION

Various antithrombogenic medical material have been developed as described above. In particular, the antithrombogenic material that the applicant of this application developed can be dissolved in an alcohol solvent to be easily utilized as a coating agent for a medical device that is contacted with blood.

The inventors of the present invention, however, found that the antithrombogenic material that the applicant developed sometimes becomes cloudy. When an antithrombogenic material becomes cloudy, the visibility of symbols, numbers, letters or the like printed on the surface of a medical device is decreased and an adhered foreign object may be hardly found. In addition, when a transparent medical device is coated with such a cloud material, the transparency is decreased. Further, the solubility of such a cloud material in a solvent may be decreased.

The objective of the present invention under such circumstances is to provide a method for producing an antithrombogenic material having less white turbidity, a method for producing a medical device coated with the antithrombogenic material produced by the method, and a medical device having less white turbidity.

### MEANS FOR SOLVING THE PROBLEMS

The inventors of the present invention made extensive studies to solve the above-described problems. As a result, the inventors completed the present invention by finding that an oligomer of silicone (meth)acrylate used as a raw material causes the white turbidity of an antithrombogenic material and the white turbidity of an antithrombogenic material can be suppressed by using an appropriate raw material silicone (meth)acrylate.

Hereinafter, the present invention is described.
[1] A method for producing an antithrombogenic material, comprising the step of copolymerizing an alkyl (meth)acrylate represented by the following formula (I), a silicone (meth)acrylate represented by the following formula (II), and an alkoxypolyethylene glycol (meth)acrylate represented by the following formula (III),
   wherein the weight average molecular weight of the silicone (meth)acrylate is 1450 or less. 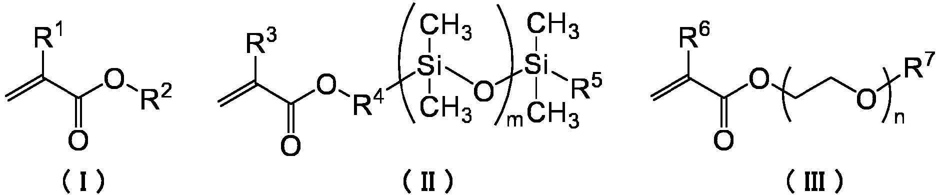 wherein
   R¹ is a hydrogen atom or a methyl group,
   R² is a C₆₋₂₀ alkyl group, a C₆₋₁₂ aromatic hydrocarbon group, or a C₆₋₁₂ aromatic hydrocarbon-C₁₋₆ alkyl group,
   R³ is a hydrogen atom or a methyl group,
   R⁴ is a C₁₋₆ alkane-diyl group,
   R⁵ is a C₁₋₆ alkyl group,
   R⁶ is a hydrogen atom or a methyl group,
   R⁷ is a C₁₋₆ alkyl group,
   m is an integer of 1 or more and 50 or less,
   n is an integer of 2 or more and 10 or less.
[2] The method according to the above [1], wherein the weight average molecular weight is more than 1000.
[3] The method according to the above [1] or [2], wherein 1.5 times or more by mole and 2 times or less by mole of the alkyl (meth)acrylate is used to the alkoxypolyethylene glycol (meth)acrylate.
[4] The method according to any one of the above [1] to [3], wherein 0.01 times or more by mole and 0.1 times or less by mole of the silicone (meth)acrylate is used to the alkoxypolyethylene glycol (meth)acrylate.
[5] The method according to the above [2], further comprising the step of concentrating a reaction mixture of the copolymerizing step under reduced pressure.
[6] A method for producing a medical device,
   wherein the medical device is contacted with blood,
   the method comprising the steps of:
      copolymerizing an alkyl (meth)acrylate represented by the formula (I), a silicone (meth)acrylate represented by the formula (II), and an alkoxypolyethylene glycol (meth)acrylate represented by the formula (III) to produce a copolymer, and
      applying the copolymer or a solution thereof on a surface of a medical device,
      wherein the weight average molecular weight of the silicone (meth)acrylate represented by the formula (II) is 1450 or less.
[7] An antithrombogenic material,
   comprising a copolymer of an alkyl (meth)acrylate represented by the formula (I), a silicone (meth)acrylate represented by the formula (II), and an alkoxypolyethylene glycol (meth)acrylate represented by the formula (III),
   wherein turbidity is 10 or less.
[8] An antithrombogenic material,
   comprising a structural unit represented by the following formula (IV), a structural unit represented by the following formula (V), and a structural unit represented by the following formula (VI),
   wherein turbidity is 10 or less. 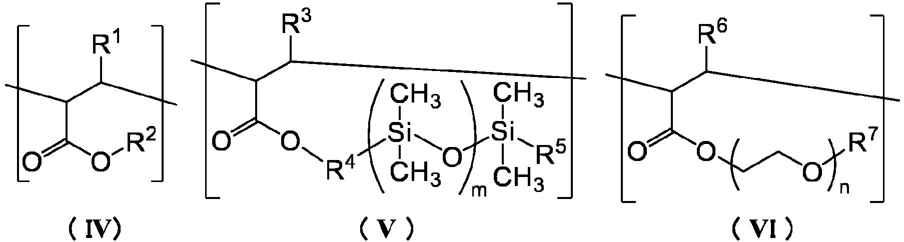 wherein R¹ to R⁷, m and n have the same meanings as the above.
[9] Use of a composition comprising a copolymer of an alkyl (meth)acrylate represented by the formula (I), a silicone (meth)acrylate represented by the formula (II), and an alkoxypolyethylene glycol (meth)acrylate represented by the formula (III), wherein turbidity is 10 or less, as an antithrombogenic material.
[10] Use of a composition comprising a copolymer comprising a structural unit represented by the formula (IV), a structural unit represented by the formula (V), and a structural unit represented by the formula (VI), wherein turbidity is 10 or less, as an antithrombogenic material.
[11] A method for improving the antithrombogenicity of a medical device,
   wherein the medical device is contacted with blood,
   the method comprising the steps of:
      copolymerizing an alkyl (meth)acrylate represented by the formula (I), a silicone (meth)acrylate represented by the formula (II), and an alkoxypolyethylene glycol (meth)acrylate represented by the formula (III) to produce a copolymer, and
      applying the copolymer or a solution thereof to a surface of a medical device,
      wherein the weight average molecular weight of the silicone (meth)acrylate represented by the formula (II) is 1450 or less.
[12] A method for improving the antithrombogenicity of a medical device,
   wherein the medical device is contacted with blood,
   the method comprising the step of applying a copolymer comprising a structural unit represented by the formula (IV), a structural unit represented by the formula (V), and a structural unit represented by the formula (VI) or a solution thereof to a surface of a medical device,
   wherein turbidity of the copolymer of the solution thereof is 10 or less.

The "C₁₋₆ alkyl group" means a monovalent linear, branched or cyclic saturated aliphatic hydrocarbon group having a carbon number of 1 or more and 6 or less. An example of the group includes methyl, ethyl, n-propyl, isopropyl, cyclopropyl, n-butyl, isobutyl, s-butyl, t-butyl, cyclobutyl, n-pentyl, cyclopentyl, n-hexyl and cyclohexyl. The R⁵ is preferably a C₂₋₆ alkyl group, more preferably a C₃₋₅ alkyl group, the most preferably n-butyl, and the R⁷ is preferably a C₁₋₄ alkyl group, more preferably a C₁₋₂ alkyl group, the most preferably methyl.

The "C₆₋₂₀ alkyl group" means a monovalent linear or branched saturated aliphatic hydrocarbon group having a carbon number of 6 or more and 20 or less. An example thereof includes n-hexyl, 2-methylpentyl, 2-ethylbutyl, cyclohexyl, n-heptyl, 2-methylhexyl, 2-ethylpentyl, n-octyl, 2-methylheptyl, 2-ethylheptyl, n-octyl, 2-methylheptyl, 2-ethylhexyl, n-nonyl, 2-methyloctyl, n-decyl, 2-methylnonyl, 2-ethyloctyl, n-lauryl, 2-methyldecyl, 2-ethylnonyl, n-myristyl, 2-methyllauryl, 2-ethyldecyl, n-palmityl, 2-methylmyristyl, 2-ethyllauryl, n-stearyl, 2-methylpalmityl and 2-ethylmyristyl. The C₆₋₂₀ alkyl group is preferably a C₈₋₁₂ alkyl group and more preferably a branched C₈₋₁₂ alkyl group.

The "C₆₋₁₂ aromatic hydrocarbon group" means a monovalent aromatic hydrocarbon group having a carbon number of 6 or more and 12 or less. An example thereof includes phenyl, naphthyl, indenyl and biphenyl, and the group is preferably phenyl.

The "C₆₋₁₂ aromatic hydrocarbon-C₁₋₆ alkyl group" means a C₁₋₆ alkyl group substituted with a C₆₋₁₂ aromatic hydrocarbon. An example thereof includes benzyl, phenethyl, phenylpropyl, naphthylmethyl, naphthylethyl and biphenylmethyl, and the group is preferably benzyl.

The "C₁₋₆ alkane-diyl group" means a divalent linear or branched saturated aliphatic hydrocarbon group having a carbon number of 1 or more and 6 or less. An example thereof includes methylene, ethylene, methylmethylene, n-propylene, methylethylene, n-butylene, methylpropylene, dimethylethylene, n-pentylene and n-hexylene, and the group is preferably a C₁₋₄ alkane-diyl group, more preferably a C₃₋₄ alkane-diyl group and the most preferably n-propane-diyl.

The "m" is preferably 2 or more, more preferably 5 or more, even more preferably 8 or more, and preferably 25 or less or 20 or less, more preferably 15 or less, even more preferably 12 or less.

The "n" is preferably 8 or less, more preferably 5 or less, and even more preferably 4 or 3.

### EFFECT OF THE INVENTION

An antithrombogenic material having less white turbidity can be produced by the present invention method. The antithrombogenic material has antithrombogenicity, since the adherence of blood platelet and the adsorption of a protein on the antithrombogenic material are suppressed due to the hydrophilic part. In addition, even when the antithrombogenic material is contacted with blood, the activation of complement is inhibited due to the water-repellency. Further, since the antithrombogenic material also has a hydrophobic part, the antithrombogenic material has affinity for the hydrophobic surface of a medical device. Furthermore, since the white turbidity of the antithrombogenic material produced by the present invention method is suppressed, the antithrombogenic material does not interfere the visibility of a medical device surface and the transparency of a transparent medical device. Thus, the present invention is industrially very excellent as a technology for producing the antithrombogenic material that gives the blood compatibility of a medical device and secures the visibility of the surface thereof, since the antithrombogenic material gives a medical device not only antithrombogenicity and suppressibility of an allergic reaction caused by complement activation but also durability to the contact with blood.

### BRIEF DESCRIPTION OF THE DRAWINGS

[Figure 1] Figure 1 is a graph to show the relationship between the weight average molecular weight of raw material poly(dimethylsiloxane) methacrylate and the turbidity of the copolymer of the monomer.
[Figure 2] Figure 2 are ¹H NMR charts of raw material poly(dimethylsiloxane) methacrylate for the copolymer having white turbidity and the transparent copolymer.

### MODE FOR CARRYING OUT THE INVENTION

The method for producing an antithrombogenic material and the method for producing a medical device that is contacted with blood are hereinafter described, and the present invention is not restricted to the following specific embodiments.

### 1. Polymerization reaction step

The alkyl (meth)acrylate represented by the formula (I), the silicone (meth)acrylate represented by the formula (II) and having weight average molecular weight of 1450 or less, and the alkoxypolyethylene glycol (meth)acrylate represented by the formula (III) are copolymerized in this step. Hereinafter, the compound represented by the formula (x) is abbreviated as the "compound (X)" in some cases. For example, the alkyl (meth)acrylate represented by the formula (I) is abbreviated as the "alkyl (meth)acrylate (I)" in some cases.

The alkyl (meth)acrylate (I) inhibits the delamination of the copolymer from a medical device by giving hydrophobicity to the copolymer and thus improving the affinity of the copolymer for the surface of the medical device to be coated.

An example of the alkyl (meth)acrylate (I) includes a linear alkyl (meth)acrylate such as n-hexyl (meth)acrylate, n-heptyl (meth)acrylate, n-octyl (meth)acrylate, n-nonyl (meth)acrylate, n-decyl (meth)acrylate, n-lauryl (meth)acrylate, myristyl (meth)acrylate, palmityl (meth)acrylate and stearyl (meth)acrylate; a branched alkyl (meth)acrylate such as 2-methylpentyl (meth)acrylate, 2-ethylbutyl (meth)acrylate, 2-methylhexyl (meth)acrylate, 2-ethylpentyl (meth)acrylate, 2-methylheptyl (meth)acrylate, 2-ethylhexyl (meth)acrylate, 2-methyloctyl (meth)acrylate, 2-ethylheptyl (meth)acrylate, 2-methylnonyl (meth)acrylate, 2-ethyloctyl (meth)acrylate, 2-methyldecyl (meth)acrylate, 2-ethylnonyl (meth)acrylate, 2-methyllauryl (meth)acrylate, 2-ethyldecyl (meth)acrylate, 2-methylmyristyl (meth)acrylate, 2-ethyllauryl (meth)acrylate, 2-methylpalmityl (meth)acrylate and 2-ethylmyristyl (meth)acrylate; a cyclic alkyl (meth)acrylate such as cyclohexyl (meth)acrylate; phenyl (meth)acrylate and benzyl (meth)acrylate, but the alkyl (meth)acrylate (I) is particularly not restricted thereto. In terms of a cost and performance, 2-ethylhexyl (meth)acrylate and n-lauryl (meth)acrylate are preferred. In addition, a branched alkyl (meth)acrylate is preferred in terms of the affinity for the surface of a medical device.

The silicone (meth)acrylate (II) suppresses the immune response in blood caused by the recognition of a foreign substance and the activation of complement by giving water repellency to the copolymer and thus reducing the surface energy thereof. The water repellency particularly means a property of repelling water among hydrophobicity. The adsorption and the adherence of a circulating protein are inhibited, and the affinity for blood containing water as a main component is reduced and thus the activation of complement is suppressed by water repellency.

The dimethylsiloxane repeating unit "m" of the silicone (meth)acrylate (II) is preferably 1 or more and 50 or less. When the "m" is 50 or less, the viscosity of the obtained copolymer and a solution thereof does not become excessively high and thus the handling property is high. On the one hand, when "m" is 1 or more, the obtained copolymer and a solution thereof can be prevented from not being fixed on the surface of a medical device to be coated due to excessively low viscosity. The "m" is preferably 2 or more, more preferably 5 or more, and preferably 40 or less or 30 or less, more preferably 20 or less or 15 or less.

The silicone (meth)acrylate (II) having weight average molecular weight of 1450 or less is used in the present invention. The inventors of the present invention experimentally found that when the weight average molecular weight is 1450 or less, white turbidity in the obtained copolymer and a solution thereof can be sufficiently suppressed. Specifically, the turbidity of the copolymer and the solution thereof can be 10 or less. The weight average molecular weight is preferably 1400 or less, more preferably 1300 or less, and even more preferably 1200 or less. On the one hand, when the weight average molecular weight is excessively small, the reaction mixture for the polymerization tends to become foamy and cannot be concentrated under reduced pressure in some cases since the polymerization reaction mixture overflows out of a reaction vessel during the concentration under reduced pressure. The weight average molecular weight is therefore preferably more than 1000, more preferably 1050 or more and even more preferably 1100 or more.

The average molecular weight of a polymer molecule is generally classified into number average molecular weight and weight average molecular weight. Number average molecular weight is a total of the numbers calculated by dividing the product of molecular weight Mᵢ and the number Nᵢ of the molecule having the molecular weight by the molecular number Nᵢ. Number average molecular weight means a simple average of the molecular weight of one polymer molecule chain in a group of polymer molecules and corresponds to a mass per 1 mole (g/mol). Weight average molecular weight is a total of the numbers calculated by dividing Mᵢ²×Nᵢ by Mᵢ×Nᵢ. In other words, weight average molecular weight corresponds to a total of mass calculated by multiplying the molecular weight of each polymer molecule by a weight fraction thereof. Weight average molecular weight can be said to have a high correlation with the physical properties of the polymer aggregate, since the value of weight average molecular weight becomes larger than that of number average molecular weight in the case where the proportion of polymer molecules having large molecular weight, which significantly contributes to the physical properties, is large. An example of a method for measuring weight average molecular weight includes end-group analysis, osmotic pressure method, vapor pressure osmometry, vapor pressure depression, freezing point depression method, ebullioscopy and gel permeation chromatography (GPC) method, and commonly used gel permeation chromatography (GPC) method is used in the present invention in terms of easiness of operation.

Silicone has the advantage of being able to show stable characteristics in a wide range of temperature, since silicone has excellent heat resistance and cold resistance and has low glass transition temperature (Tg) as can be seen from the basic structure thereof. In addition, silicone has the advantage of resistance to acid and alkali and high chemical stability due to high binding energy. Further, silicone is preferably utilized as a raw material of the (meth)acrylate copolymer according to the present invention, since silicone is excellent in the copolymerization property with a (meth)acrylate monomer. Silicone (meth)acrylate is also recognized as a material with high safety for a human body. For example, silicone has been used as a material for a contact lens in recent years. Thus, even if the content amount of the silicone (meth)acrylate (II) in the antithrombogenic material is excessively large, a problem may not be caused. But the ratio of the silicone (meth)acrylate (II) to the total of the alkyl (meth)acrylate (I) and the silicone (meth)acrylate (II) may be 50 mass% or less in comprehensive consideration of the performance, quality and a cost, since the raw material price of the silicone (meth)acrylate (II) is relatively high under the current circumstances. The ratio is preferably 40 mass% or less and more preferably 35 mass% or less. On the one hand, the ratio of the silicone (meth)acrylate (II) to the total of the alkyl (meth)acrylate (I) and the silicone (meth)acrylate (II) is preferably 0.1 mass% or more, more preferably 0.5 mass% or more and even more preferably 1.0 mass% or more in terms of the long-term stability of the copolymer.

The alkoxypolyethylene glycol (meth)acrylate (III) suppresses the adherence of blood platelet and subsequent activation thereof in blood, and inhibits the adsorption of a protein by giving hydrophilicity to the copolymer.

The number "n" of the repeating unit of the ethylene oxide in the alkoxypolyethylene glycol (meth)acrylate (III) is preferably 2 or more and 10 or less. When the "n" is 2 or more, the hydrophilicity of the copolymer is sufficient. When the "n" is 10 or less, the elution of the copolymer to blood and the desorption of the copolymer from a medical device can be sufficiently suppressed. The "n" is more preferably 5 or less and even more preferably 3 or 4.

An example of the alkoxypolyethylene glycol (meth)acrylate (III) specifically includes methoxy diethylene glycol (meth)acrylate, methoxy triethylene glycol (meth)acrylate, methoxy tetraethylene glycol (meth)acrylate, methoxy pentaethylene glycol (meth)acrylate, methoxy hexaethylene glycol (meth)acrylate, methoxy heptaethylene glycol (meth)acrylate, methoxy octaethylene glycol (meth)acrylate, methoxy nonaethylene glycol (meth)acrylate and methoxy decaethylene glycol (meth)acrylate.

The usage amounts of the alkoxypolyethylene glycol (meth)acrylate (III) and the alkyl (meth)acrylate (I) may be appropriately adjusted. For example, the molar ratio of the alkyl (meth)acrylate (I) to 1 mole of the alkoxypolyethylene glycol (meth)acrylate (III) may be adjusted to 0.5 or more and 2.5 or less. When the molar ratio is included in the above-described range, the balance between the hydrophilic part and the hydrophobic part of the antithrombogenic material is good. As a result, the affinity of the antithrombogenic material for the surface of a medical device can be ensured and thus the elution to blood and the coating unevenness of the antithrombogenic material can be suppressed with inhibiting the adsorption of blood platelet and a circulating protein on the surface of a medical device in blood. The ratio is preferably 1 or more, more preferably 1.2 or more or 1.4 or more, even more preferably 1.5 or more, and preferably 2.2 or less, more preferably 2 or less.

The usage amounts of the alkoxypolyethylene glycol (meth)acrylate (III) and the silicone (meth)acrylate (II) may be also appropriately adjusted. For example, the molar ratio of the silicone (meth)acrylate (II) to 1 mole of the alkoxypolyethylene glycol (meth)acrylate (III) may be adjusted to 0.001 or more and 1 or less. When the molar ratio is included in the above-described range, not only the adsorption of blood platelet and a circulating protein on the surface of a medical device but also the activation of complement due to the antithrombogenic material can be suppressed. As a result, an excessive immune reaction can be inhibited. The molar ratio is preferably 0.005 or more, more preferably 0.01 or more, and preferably 0.5 or less, more preferably 0.1 or less.

The alkyl (meth)acrylate (I), the silicone (meth)acrylate (II) and the alkoxypolyethylene glycol (meth)acrylate (III) are copolymerized in a solvent in this step. The copolymerization reaction itself for producing the antithrombogenic material of the present invention is particularly not restricted. A publicly known method such as radical polymerization, ion polymerization, photopolymerization and polymerization using a macromer can be used, and radical polymerization using a radical initiator is preferred.

An example of a solvent used for the copolymerization includes an alcohol solvent such as methanol, ethanol and 2-propanol; an ester solvent such as ethyl acetate; an aromatic hydrocarbon solvent such as toluene and benzene; a ketone solvent such as methyl ethyl ketone; and water. The solvent is preferably ethyl acetate, methanol and ethanol in terms of the ability to dissolve the monomer and the produced copolymer therein and accessibility. In addition, a plurality of the above solvent may be mixed to be used.

The usage amount of the solvent may be appropriately adjusted. For example, 0.3 times or more by mass and 10 times or less by mass of the solvent to the total amount of the monomer may be used. The ratio is preferably 0.5 times or more by mass and 5 times or less by mass.

A peroxide radical initiator and an azo radical initiator, which are generally used for radical polymerization, are used as a radical initiator. An example of the peroxide radical initiator includes an inorganic peroxide such as potassium persulfate, ammonium persulfate and hydrogen peroxide; and an organic peroxide such as benzoyl peroxide, t-butyl hydroperoxide and cumene peroxide. An example of the azo radical initiator includes 2,2'-azobis(isobutyronitrile), 2,2'-azobis(2-amidinopropane) dihydrochloride, dimethyl 2,2'-azobis(butyrate) and dimethyl 2,2'-azobis(2-methylpropionate). In addition, a redox initiator, which is a combination of a peroxide initiator and a reducing agent, can be also used.

The usage amount of a radical initiator may be appropriately adjusted. For example, 0.01 mass% or more and 1 mass% or less of a radical initiator to the total amount of the monomer may be used. The ratio is preferably 0.05 mass% or more, and preferably 0.5 mass% or less and more preferably 0.3 mass% or less.

Specifically, for example, each monomer, a polymerization solvent and a radical initiator are added to a reaction device equipped with a reflux tower and capable of stirring, the gas phase is substituted with nitrogen, and then the mixture is heated to start polymerization. The polymerization proceeds by maintaining the temperature during a given time. The molecular weight can be controlled using a chain transfer agent during the polymerization. An example of the chain transfer agent used for controlling the molecular weight during the polymerization includes isopropyl alcohol, phosphorus acid, hypophosphorous acid and the like in addition to a thiol compound having a high boiling point, such as dodecylmercaptan, thiomalic acid and thioglycolic acid.

For example, the temperature for the polymerization is preferably adjusted to about 10-hr half-life temperature of the radical initiator depending on the kind of the solvent and the radical initiator. Specifically, when the above-described radical initiator is used, the temperature may be adjusted to 20°C or higher and 90°C or lower. The temperature is preferably 30°C or higher and more preferably 40°C or higher. The time for the polymerization reaction may be also appropriately adjusted. For example, the polymerization reaction may be carried out until it is confirmed by chromatography or the like that any one of the monomers is consumed. Alternatively, the time may be determined by a preliminary experiment. Specifically, the time may be adjusted to 1 hour or more and 10 hours or less.

The crude (meth)acrylate copolymer can be obtained by removing the solvent after the polymerization reaction. When the silicone (meth)acrylate (II) having an excessively small weight average molecular weight is used in the polymerization reaction for inhibiting white turbidity of the antithrombogenic material, the reaction mixture may not be concentrated under reduced pressure in some cases, since the reaction mixture may become foamy and spill out from a vessel by the concentration under reduced pressure. Thus, such forming can be inhibited more surely and the reaction mixture can be successfully concentrated under reduced pressure by using the silicone (meth)acrylate (II) having weight average molecular weight of more than 1000.

The copolymer can be purified by removing the solvent, stirring the obtained crude (meth)acrylate copolymer in a poor solvent, and then removing the poor solvent. The purity of the (meth)acrylate copolymer can be improved by repeating the purification treatment 1 time or more and several times or less. The thus obtained copolymer may be dried.

Since the (meth)acrylate copolymer is produced by copolymerizing a hydrophilic monomer and a hydrophobic monomer in the present invention, the (meth)acrylate copolymer has both of a hydrophilic property and a hydrophobic property. Thus, there are a hydrophilic monomer (methoxypolyethylene glycol (meth)acrylate) and hydrophobic monomers (silicone (meth)acrylate and alkyl (meth)acrylate) as unreacted monomers and the (meth)acrylate copolymer in the solution after the polymerization reaction. For example, the copolymer can be purified by adding the solution of the copolymer dropwise to a solvent that can dissolve the hydrophilic monomer to remove the hydrophilic monomer and then using a solvent that can dissolve the hydrophobic monomer in order to isolate the water-insoluble (meth)acrylate copolymer from the mixture. In addition, the (meth)acrylate copolymer can be efficiently collected by using a poor solvent for reprecipitation. Such a poor solvent can be prepared by mixing an alcohol and water in the specific ratio. Further, the (meth)acrylate copolymer can be purified by repeating the following procedure: a poor solvent that is a mixed liquid of an alcohol and water in the specific ratio is added to the water-insoluble (meth)acrylate copolymer in the solution after the polymerization reaction, the mixture is stirred at a certain temperature to precipitate the (meth)acrylate copolymer, the precipitate is collected by decantation, and a washing liquid is added to the precipitate.

A poor solvent that does not dissolve the copolymer and that can dissolve both of the hydrophilic monomer and the hydrophobic monomer is preferably used as a poor solvent used for purifying the copolymer in the present invention.

An alcohol used for reprecipitation treatment in the present invention is preferably a C₁₋₁₀ alcohol, more preferably a C₁₋₇ alcohol, and even more preferably a C₁₋₄ alcohol. A specific example of such an alcohol includes methanol, ethanol, 1-propanol, 2-propanol, 1-butanol, 2-butanol, 2-methoxy-1-propanol and t-butanol. Methanol, ethanol, 1-propanol and 2-propanol are more preferred, since the alcohols can be dried under low temperature for a short time.

The usage amount of the poor solvent may be appropriately adjusted as long as the copolymer can be sufficiently purified, and for example, a volume ratio of the poor solvent to the crude (meth)acrylate copolymer is preferably adjusted to 1 or more and 20 or less and more preferably 5 or less.

The temperature for the purification of the crude (meth)acrylate copolymer according to the present invention is preferably 30°C or higher and 60°C or lower and more preferably 40°C or higher and 60°C or lower. When the temperature for the purification is included in the above-described range, the viscosity of the crude (meth)acrylate copolymer is reduced by heating and thus the copolymer can be easily separated from the poor solvent. As a result, the collection rate of the (meth)acrylate copolymer can be maximally increased.

The collection rate of the (meth)acrylate copolymer after the purification treatment is preferably 20 mass% or more and 90 mass% or less in the present invention. When the collection rate is 20 mass% or more, the production efficiency may be relatively high. When the collection rate is 90 mass% or less, the unreacted monomer may be sufficiently prevented from being mixed in the copolymer. When the collection rate is adjusted within the above-described range, a small amount of the copolymer is lost or abandoned unavoidably to prevent the contamination by the unreacted monomer. Such a sacrifice is unavoidable, since the copolymer has both of hydrophilic and hydrophobic characteristics and is applied to a medical device.

The amount of the remaining monomer, i.e. the amount of the monomer unreacted in the polymerization, is critical in the present invention, since the amount relates to safety. Though the standard set in the guideline for the elution from a medical device should be met by reducing the amount of the remaining monomer in the antithrombogenic material, it was surprising that the antithrombogenic material can be adhered and retained on the surface of a medical device by reducing the amount of the remaining monomer to very small level as 4,000 ppm or less. In addition, a collection rate is described as the ratio of the obtained copolymer amount to the amount of the used monomer.

The water-insoluble (meth)acrylate copolymer having the content amount of the unreacted monomer of 4,000 ppm or less can be collected with high collection rate of 30 mass% or more by carrying out the above-described purification treatment once. If it is necessary, the purification treatment may be carried out 2 times or more and 8 times or less. When the content amounts of the unreacted monomer, oligomer and polymerization residue in the copolymer is large, the impurities may be eluted in blood to be a substance responsible for a shock disease or the like of a patient. Most of such a substance can be removed by the purification treatment, and the content amount thereof is preferably reduced to 3,000 ppm or less in terms of the safety of a patient. The content amount is more preferably 2,000 ppm or less and particularly preferably 1,000 ppm or less.

When the purified copolymer is used for giving antithrombogenicity to a medical device, the solvent should be removed by drying. For example, the copolymer is dried at 60°C under reduced pressure such as 1 Torr or less; nonetheless, if the copolymer cannot be sufficiently dried, the copolymer may be continuously dried under reduced pressure.

The copolymer produced by copolymerizing the alkyl (meth)acrylate (I), the silicone (meth)acrylate (II) and the alkoxypolyethylene glycol (meth)acrylate (III) in the present invention, in other words, the copolymer having the alkyl (meth)acrylate structural unit (IV), the silicone (meth)acrylate structural unit (V) and the alkoxypolyethylene glycol (meth)acrylate structural unit (VI), is durable against blood contact and is preferably a viscous liquid at room temperature. The term, "durable against blood contact" means in this disclosure that when the (meth)acrylate copolymer is immersed in the alcohol immersion treatment liquid described later at room temperature for 16 hours, the specified amount of the (meth)acrylate copolymer remains to show antithrombogenicity. If the specified amount of the (meth)acrylate copolymer remains in the case where the copolymer is immersed in the alcohol immersion treatment liquid at room temperature for 16 hours, it can be judged that the copolymer shows sufficient antithrombogenicity even in the case where the copolymer is contacted with blood at 37°C for 30 days. In addition, the copolymer has the advantage of being less likely to be dissolved into blood in the case where a medical device is coated with the copolymer to be used, since the copolymer is liquid but viscous at room temperature.

In addition, the copolymer produced by the present invention method hardly produces white turbidity and is excellent in transparency. Specifically, the turbidity of the copolymer and the antithrombogenic material according to the present invention is low as 10 or less. The "turbidity" means the turbidity measured in accordance with the method provided by Testing methods for industrial water of JIS K0101:2017 and The Japanese Pharmacopoeia, Eighteenth Edition. Specifically, a calibration curve is prepared by measuring absorption at 660 nm of a turbidity standard solution, the absorption at 660 nm of the copolymer and the antithrombogenic material according to the present invention is measured, and the turbidity of the copolymer and the antithrombogenic material according to the present invention may be determined from the measured value using the calibration curve. The turbidity is preferably 8 or less or 6 or less, more preferably 5 or less, 4 or less or 3 or less, and even more preferably 2 or less, 1.5 or less or 1 or less.

An example of the method for confirming the resistance to the elution of the antithrombogenic material according to the present invention to blood includes an alcohol immersion treatment at room temperature. An example of the alcohol preferably includes a mixed solvent of methanol and ethanol. For example, the continuous antithrombogenicity can be evaluated by immersing the antithrombogenic material in a mixed solvent of methanol/ethanol=80/20 by mass for 16 hours, since the elution capability of the mixed solvent is slightly stronger than that of blood.

The (meth)acrylate copolymer of the present invention is not dissolved in methanol but is dissolved in ethanol. The resistance to the contact to blood at 37°C for 30 days, i.e. continuous antithrombogenicity, can be confirmed by using a mixed solvent of methanol and ethanol in the specific ratio as an alcohol immersion treatment liquid even in a short time as 16 hours. The mass ratio of methanol and ethanol in the alcohol immersion treatment liquid is preferably methanol:ethanol=90 to 60 : 10 to 40 and more preferably 90 to 70 : 10 to 30. When a blood clot is small, in other words, the number of an evaluation sheet on which a blood clot adheres is less than 1 out of 10 sheets after immersion in a solvent and 0.1 µg/cm² or more of the (meth)acrylate copolymer remains in the test described later using an evaluation sheet, it can be judged that the copolymer have sufficient resistance.

A blood coagulation test can be exemplified as one of the methods for evaluating blood compatibility of the copolymer in the present invention. Specifically, the reaction in which fibrin in plasma gels by a calcium ion to form fibrin gel is used in the test. Blood compatibility of the polymer can be evaluated by confirming whether blood clot adheres on a sample or not in blood plasma in which a calcium ion is added after the sample is immersed in water. For example, an evaluation sheet is prepared by immersing the half-plane of a polycarbonate sheet in an ethanol solution of the copolymer and drying the sheet. A test blood solution is added to the evaluation sheet, and it is confirmed whether blood clot adheres on the sheet or not. When the number of the evaluation sheet on which blood clot adheres is 4 or less out of 10, blood compatibility can be regarded as good.

An example of the (meth)acrylate copolymer of the present invention specifically includes silicone (meth)acrylate-methoxydiethylene glycol (meth)acrylate copolymer, silicone (meth)acrylate-methoxytriethylene glycol (meth)acrylate copolymer, silicone (meth)acrylate-methoxytetraethylene glycol (meth)acrylate copolymer, silicone (meth)acrylate-n-hexyl (meth)acrylate-methoxydiethylene glycol (meth)acrylate copolymer, silicone (meth)acrylate-n-hexyl (meth)acrylate-methoxytriethylene glycol (meth)acrylate copolymer, silicone (meth)acrylate-n-hexyl (meth)acrylate-methoxytetraethylene glycol (meth)acrylate copolymer, silicone (meth)acrylate-cyclohexyl (meth)acrylate-methoxydiethylene glycol (meth)acrylate copolymer, silicone (meth)acrylate-cyclohexyl (meth)acrylate-methoxytriethylene glycol (meth)acrylate copolymer, silicone (meth)acrylate-cyclohexyl (meth)acrylate-methoxytetraethylene glycol (meth)acrylate copolymer, silicone (meth)acrylate-phenyl (meth)acrylate-methoxydiethylene glycol (meth)acrylate copolymer, silicone (meth)acrylate-phenyl (meth)acrylate-methoxytriethylene glycol (meth)acrylate copolymer, silicone (meth)acrylate-phenyl (meth)acrylate-methoxytetraethylene glycol (meth)acrylate copolymer, silicone (meth)acrylate-n-octyl (meth)acrylate-methoxydiethylene glycol (meth)acrylate copolymer, silicone (meth)acrylate-n-octyl (meth)acrylate-methoxytriethylene glycol (meth)acrylate copolymer, silicone (meth)acrylate-n-octyl (meth)acrylate-methoxytetraethylene glycol (meth)acrylate copolymer, silicone (meth)acrylate-2-ethylhexyl (meth)acrylate-methoxydiethylene glycol (meth)acrylate copolymer, silicone (meth)acrylate-2-ethylhexyl (meth)acrylate-methoxytriethylene glycol (meth)acrylate copolymer, silicone (meth)acrylate-2-ethylhexyl (meth)acrylate-methoxytetraethylene glycol (meth)acrylate copolymer, silicone (meth)acrylate-lauryl (meth)acrylate-methoxydiethylene glycol (meth)acrylate copolymer, silicone (meth)acrylate-lauryl (meth)acrylate-methoxytriethylene glycol (meth)acrylate copolymer, silicone (meth)acrylate-lauryl (meth)acrylate-methoxytetraethylene glycol (meth)acrylate copolymer, silicone (meth)acrylate-n-nonyl (meth)acrylate-methoxydiethylene glycol (meth)acrylate copolymer, silicone (meth)acrylate-n-nonyl (meth)acrylate-methoxytriethylene glycol (meth)acrylate copolymer, silicone (meth)acrylate-n-nonyl (meth)acrylate-methoxytetraethylene glycol (meth)acrylate copolymer, silicone (meth)acrylate-n-decyl (meth)acrylate-methoxydiethylene glycol (meth)acrylate copolymer, silicone (meth)acrylate-n-decyl (meth)acrylate-methoxytriethylene glycol (meth)acrylate copolymer, silicone (meth)acrylate-n-decyl (meth)acrylate-methoxytetraethylene glycol (meth)acrylate copolymer, silicone (meth)acrylate-stearyl (meth)acrylate-methoxydiethylene glycol (meth)acrylate copolymer, silicone (meth)acrylate-stearyl (meth)acrylate-methoxytriethylene glycol (meth)acrylate copolymer, silicone (meth)acrylate-stearyl (meth)acrylate-methoxytetraethylene glycol (meth)acrylate copolymer, silicone (meth)acrylate-lauryl (meth)acrylate-methoxydiethylene glycol (meth)acrylate copolymer, silicone (meth)acrylate-lauryl (meth)acrylate-methoxytriethylene glycol (meth)acrylate copolymer, silicone (meth)acrylate-lauryl (meth)acrylate-methoxytetraethylene glycol (meth)acrylate copolymer, silicone (meth)acrylate-myristyl (meth)acrylate-methoxydiethylene glycol (meth)acrylate copolymer, silicone (meth)acrylate-myristyl (meth)acrylate-methoxytriethylene glycol (meth)acrylate copolymer and silicone (meth)acrylate-myristyl (meth)acrylate-methoxytetraethylene glycol (meth)acrylate copolymer. In addition, an example of the copolymer according to the present invention includes a (meth)acrylate copolymer of the alkyl (meth)acrylate (I) : the silicone (meth)acrylate (II) : the methoxy polyethylene glycol (meth)acrylate (III) = 80 to 20 : 10 to 0.01 : 10 to 79.99 by molar ratio, but the copolymer is not restricted thereto. The units of the alkyl (meth)acrylate (I) and the silicone (meth)acrylate (II) as hydrophobic (meth)acrylate suppress the elution of the copolymer into blood, and improves the resistance to blood and affinity for the surface of a medical device. The unit of the alkoxypolyethylene glycol (meth)acrylate (III) as hydrophilic (meth)acrylate suppresses the adhesion of blood platelet and a protein. The above-described molar ratio is, therefore, preferably 80 to 50 : 5 to 0.01 : 15 to 49.99, more preferably 77 to 55 : 5 to 0.01 : 18 to 44.99, and even more preferably 73 to 57 : 5 to 0.01 : 22 to 42.99. The ratios of the structural unit of each acrylate in the copolymer of the present invention can be determined on the basis of, for example, the peak intensity ratio specific to each acrylate in the analysis data of the copolymer by nuclear magnetic resonance (NMR) or mass spectrometry.

The weight average molecular weight of the copolymer is particularly not restricted and is preferably 50,000 or more and 1,500,000 or less. When the weight average molecular weight is 50,000 or more, the elution into blood can be sufficiently suppressed and the strength and the stability of the coating film can be ensured more surely. When the weight average molecular weight is 1,500,000 or less, the workability for coating a medical device is sufficiently improved. The weight average molecular weight is more preferably 1,000,000 or less and even more preferably 500,000 or less.

The reduced viscosity (ηsp/c) of the (meth)acrylate copolymer according to the present invention is preferably 0.18 dl/g or more and 3.00 dl/g or less. When the antithrombogenic material having the viscosity included in the above range is used for coating a medical device such as a heart-lung machine line and a catheter, the adherence of the copolymer on a medical device is excellent and continuous antithrombogenicity for a long time becomes possible. The reduced viscosity is more preferably 0.18 dl/g or more, and more preferably 1.50 dl/g or less, even more preferably 0.50 dl/g or less.

The antithrombogenic material of the present invention may be any one of a random copolymer, a block copolymer and a graft copolymer. The (meth)acrylate copolymer of the present invention may be a copolymer in which each monomer is alternately arranged, and may be also a copolymer composed of a segment or a block of a hydrophobic monomer and a segment or a block of a hydrophilic monomer on the basis of analysis data of a total amount. The segment or block of a hydrophobic monomer may possibly serve to fix the segment or block of a hydrophilic monomer to constitute a complicated structure such as a microphase-separated structure and a mosaic pattern-like structure. The elution of the segment or block of the hydrophilic monomer of the copolymer can be suppressed anyway by slightly increasing the amount of the hydrophobic monomer, though the size of the copolymer's molecular weight and the kind and the characteristics of the hydrophilic monomer may have some effect. In addition, the affinity for a hydrophobic medical device may be improved and the copolymer may successfully adhere on a medical device as a coating layer by slightly increasing the hydrophobic segment. The copolymer is a high molecular material having good affinity for a living body, though whether the copolymer has the segment or not and the behavior by the affinity of the segment cannot be accurately evaluated on the basis of a technical ground under the current conditions.

A homopolymer of the alkoxypolyethylene glycol (meth)acrylate (III) is excellent in blood compatibility due to high hydrophilicity but have the problem of gradual elution due to water solubility in the case where the homopolymer is contacted with blood or the like for a long time. The inventors of the present invention tried to develop a material that is excellent in blood compatibility and can be also used for a long time. As a result, the inventors found that the problem can be solved by the copolymer having appropriate hydrophobicity for inhibiting the elution into blood or the like and the flexibility for preventing physical peeling of the coating membrane.

The copolymer of the present invention is substantially composed of two kind of monomer components that respectively have different functions at the interface. Specifically, the copolymer is composed of a hydrophilic monomer, segment or block having antithrombogenicity in blood and a function to prevent the elution into blood and a hydrophobic monomer, segment or block having affinity for a medical device and a function to adhere on a medical device. The monomers, segments or blocks that constitute the copolymer complement each other within the molecular structure, and the molecules stable to elution and dispersion may also form the bond or the structure.

The (meth)acrylate copolymer of the present invention can be preferably dissolved in any one of C₁₋₆ alcohols. The copolymer can be more preferably dissolved in a C₁₋₃ alcohol, since the copolymer can be easily dried after the coating. The phrase, "can be dissolved", means that when 1 g of the (meth)acrylate copolymer is immersed in 10 mL of the alcohol at 25°C, at least 90 mass% (meth)acrylate copolymer is dissolved at room temperature within 16 hours.

The antithrombogenic material comprising the (meth)acrylate copolymer of the present invention may comprise a substance such as an antibacterial substance. Such an antibacterial substance is particularly not restricted, and an example thereof includes an antibiotic such as ampicillin, nafcillin, amoxicillin, oxacillin, azlocillin, penicillin G, carbenicillin, penicillin V, dicloxacillin, phenethicillin, floxacillin, piperacillin, mecillinam, sulbenicillin, methicillin, ticarcillin, mezlocillin, cefaclor, cephalothin, cefadroxil, cephapirin, cefamandole, cefradine, cefatrizine, cefsulodin, cefazolin, ceftazidime, ceforanide, ceftriaxone, cefoxitin, cefuroxime, cephacetrile, latamoxef, cefalexin, amikacin, neomycin, dibekacin, kanamycin, gentamicin, netilmicin, tobramycin, amphotericin B, novobiocin, bacitracin, nystatin, clindamycin, polymyxin, colistin, rovamycine, erythromycin, streptomycin, spectinomycin, lincomycin, vancomycin, chlortetracycline, oxytetracycline, demeclocycline, rolitetracycline, doxycycline, tetracycline and minocycline; an antimycotic agent such as amphotericin B, ketoconazole, clotrimazole, miconazole, econazole, natamycin, flucytosine, nystatin and griseofulvin; a p-hydroxybenzoate ester such as isobutyl p-hydroxybenzoate, isopropyl p-hydroxybenzoate, ethyl p-hydroxybenzoate, butyl p-hydroxybenzoate and propyl p-hydroxybenzoate; a biguanide compound such as chlorhexidine; a surface-active compound such as benzethonium, benzalkonium, lauryl sulfate, alkylpolyaminoethylglycine, a fatty acid and domiphen bromide; a phenol derivative such as thymol, phenol, hexachlorophene and resorcine; a boric acid compound such as boric acid and borax; an iodine compound such as iodine, iodoform and povidone-iodine; a metal such as gold, silver, copper and mercury; a metal compound such as thimerosal, methyl bromide and silver sulfadiazine; an antibacterial pigment compound such as acrinol and methylrosanilinium; and a sulfa drug such as mafenide acetate, sulfadiazine, sulfisomidine and sulfamethoxazole. The antibacterial substance may be a salt compound such as a sodium salt, a potassium salt, a magnesium salt, a calcium salt, a hydrochloride salt, a sulfate salt and a gluconate salt. Two or more antibacterial substances may be used in combination.

The antibacterial substance is classified into a water-soluble substance and a poor water-soluble substance. An example of a water-soluble antibacterial substance includes benzalkonium chloride, povidone-iodine, penicillin G potassium salt and streptomycin sulfate. An example of a poor water-soluble antibacterial substance includes silver sulfadiazine and chlorhexidine.

### 2. Coating step

The copolymer of the present invention or a solution thereof is applied on the surface of a medical device and then dried as needed in this step. The antithrombogenic material of the present invention has functions to suppress a complement activity and the adhesion of blood platelet and a protein, resistance to blood, and affinity for a hydrophobic surface. Thus, when a medical device is coated with the antithrombogenic material of the present invention, the medical device also has the properties.

An example of a method for supporting the antithrombogenic material of the present invention on the surface of a base material such as a medical device includes a publicly known method such as a coating method, a method utilizing graft polymerization by radiation, electron beam or ultraviolet ray and a method utilizing a chemical reaction with a functional group of the base material. In particular, a coating method is practically preferred due to easy production process. For example, a medical device can be coated by dissolving the antithrombogenic material of the present invention in an organic solvent to prepare a solution, applying the solution on the surface of a base material such as medical device, and then removing the solvent. A coating method is particularly not restricted, and an applying method, a spray method, a dipping method or the like can be used. The base material after the coating is preferably dried by heating. As a result, the adhesion between the base material and the antithrombogenic material of the present invention become stronger and thus the antithrombogenic material can be fixed more solidly.

When the copolymer of the present invention is liquid under atmospheric temperature and atmospheric pressure, the copolymer may be directly used for the coating. When the copolymer of the present invention is a solid or highly viscous liquid under atmospheric temperature and atmospheric pressure, a solution or a suspension prepared by dissolving or dispersing the copolymer in a solvent may be used. An organic solvent that does not damage a medical device as a base material as much as possible may be selected to prepare a coating solution, and an example thereof includes an alcohol solvent such as methanol, ethanol, 2-propanol and n-propanol; a ketone solvent such as acetone and cyclohexanone; an aliphatic hydrocarbon solvent such as n-hexane and cyclohexane; an ether solvent such as tetrahydrofuran and 1,4-dioxane; and an amide solvent such as N,N-dimethylformamide, N,N-dimethylacetamide and N-methylpyrrolidone. In particular, methanol, ethanol and 2-propanol are more preferred, since the solvents are easily dried after the coating due to low boiling point.

The concentration of the (meth)acrylate copolymer in a solution of the antithrombogenic material according to the present invention may be appropriately adjusted and may be adjusted to, for example, 0.001 mass% or more and 10 mass% or less. When the concentration is 0.001 mass% or more, a medical device can be sufficiently coated with the antithrombogenic material of the present invention. When the concentration is 10 mass% or less, the viscosity of the solution does not become excessively high and the solution can be easily treated. The concentration is preferably 0.01 mass% or more and 5 mass% or less.

An example of a method for determining an amount of the antithrombogenic material in the coating layer includes NMR. Specifically, the antithrombogenic material is extracted from the coated base material using ethanol, the extraction liquid is dried to dryness, the residue is analyzed by NMR, and the coating amount is calculated on the basis of area of the pertinent peak. The resistance can be evaluated by comparing coating amounts before and after the coated base material is immersed in alcohol. Specifically, when an amount of the remaining (meth)acrylate copolymer after an evaluation sheet is immersed in 99.5 mass% ethanol is 3.0 µg/cm² or more, antithrombogenicity in the initial phase of the contact with blood can be regarded as sufficiently high.

A medical device of which at least a part of the surface is covered with the antithrombogenic material of the present invention has excellent antithrombogenicity. An example of a medical device includes a blood filter, a blood storage container, a blood circuit, an indwelling needle, a catheter, a guide wire, a stent, an artificial lung device, a dialyzer, an anti-adhesive material, a wound dressing material, an adhesive material for a living body tissue and a repairing material for regenerating a living body tissue. In particular, a medical device having a part to be contact with blood in an extracorporeal circulation circuit is preferred.

An example of a material for a medical device includes all of generally used materials. An example of such a material includes polyvinyl chloride, polycarbonate, polyethylene terephthalate, polyethylene, polypropylene, poly-4-methylpentene-1, thermoplastic polyether polyurethane, thermosetting polyurethane, silicone rubber such as crosslinked polydimethylsiloxane, polymethylmethacrylate, polyvinylidene fluoride, polytetrafluoroethylene, polysulfone, polyether sulfone, polyacetal, polystyrene, ABS resin, and a mixture of the resins, and metal such as stainless, titanium and aluminum. Uniform and strong coating is possible by the antithrombogenic material of the present invention regardless of a raw material, a form and surface texture to be coated, since not only the antithrombogenic material has a favorable balance of a material composition, molecular weight and viscosity but also the coating condition is adjusted to be appropriate.

When a medical material or the like is coated with the copolymer of the present invention as an antithrombogenic substance and a poor water-soluble antibacterial substance in the present invention, a very small amount of the antibacterial substance is continuously eluted and thus the antibacterial activity can be maintained for a long time. That reason is not clear. For example, the reason may be that the copolymer is water-insoluble or the water-insolubility of the copolymer and the poor water-solubility of the antibacterial substance function in a complementary way. On the one hand, when a medical material is coated with the copolymer and a water-soluble antibacterial substance, the amount of the eluted antibacterial substance becomes larger in comparison with the case of a poor water-soluble antibacterial substance, since the copolymer is water-insoluble but the antibacterial substance is water-soluble. As a result, strong antibacterial activity can be instantaneously shown but antibacterial activity cannot be maintained for a long time. For example, an indwelling vascular catheter, a transfusion tube, an artificial lung or the like as a medical device is continuously used for 1 to several days, and antibacterial activity should be maintained for a long time in such a case. In addition, when a water-soluble antibacterial substance and a poor water-soluble antibacterial substance are used in combination with the copolymer, multistep antibacterial activity can be exerted. For example, strong antibacterial activity can be exerted in early times due to the water-soluble antibacterial substance and antibacterial activity can be subsequently exerted for a long time due to the poor water-soluble antibacterial substance. When such multistep antibacterial activity is applied to an indwelling vascular catheter, indigenous bacteria in skin that are brought into a blood vessel during catheter insertion can be killed and thus the risk of infection during insertion can be reduced by eluting a water-soluble antibacterial substance in an early stage to exert strong antibacterial activity. In addition, the antibacterial activity for a long time of a poor water-soluble antibacterial substance can inhibit the growth of bacteria invading from a fixed catheter part and a catheter-inserting part and thus the risk of infection during indwelling can be reduced.

The ratio of the antibacterial substance to the mass of the antithrombogenic material is preferably 0.01 mass% or more and 70 mass% or less in the present invention. When the ratio is 0.01 mass% or more, the antibacterial activity of the antibacterial substance is exerted more surely. When the ratio is 70 mass% or less, poor appearance of a medical device after surface treatment such as the coating, the elution of an antibacterial substance within a living body, localized inflammation caused by such an elution can be suppressed more surely. The ratio is more preferably 0.05 mass% or more, even more preferably 0.1 mass% or more, and more preferably 50 mass% or less or 30 mass% or less, even more preferably 10 mass% or less. All of the surface of a medical device may be coated with the antibacterial substance, but there is preferably the antibacterial substance around an insertional part through the skin only for suppressing localized inflammation.

The (meth)acrylate copolymer of the present invention produced by copolymerizing the alkyl (meth)acrylate (I), the silicone (meth)acrylate (II) and the alkoxypolyethylene glycol (meth)acrylate (III) can be preferably used as a blood-compatible material, since the copolymer has a favorable balance of hydrophilicity and hydrophobicity. In addition, the copolymer can be preferably used as a material for treating a medical device, since the adsorption and the adhesion of blood platelet, a protein in blood or the like can be suppressed. Further, one of the (meth)acrylate copolymer of the present invention can be used, or a plurality of the (meth)acrylate copolymer can be used in combination.

When a medical device treated with the antithrombogenic material of the present invention is contacted with blood, the highly hydrophilic alkoxypolyethylene glycol (meth)acrylate (III) may be exposed on the surface to exert antithrombogenicity and the hydrophobic alkyl (meth)acrylate (I), the silicone (meth)acrylate (II) and (meth)acrylate may prevent the medical device from contacting with blood by remaining adjacent to the base material.

The present application claims the benefit of the priority date of Japanese patent application No. 2023-205501 filed on December 5, 2023. All of the contents of the Japanese patent application No. 2023-205501 filed on December 5, 2023, are incorporated by reference herein.

### EXAMPLES

Hereinafter, the present invention is described in more detail with Examples. The present invention is however not restricted to the following Examples in any way, and it is possible to work the present invention according to the Examples with an additional appropriate change within the range of the above descriptions and the following descriptions. Such a changed embodiment is also included in the technical scope of the present invention.

### Example 1

### (1) Copolymerization reaction

Poly(dimethylsiloxane) methacrylate (PDMSMA) having various weight average molecular weight were obtained. The weight average molecular weight of each PDMSMA are described in Table 1.

Azobisisobutyronitrile (AIBN) manufactured by FUJIFILM Wako Pure Chemical (1.23 g) was added to methoxytriethylene glycol acrylate (MTEGA) manufactured by SHIN-NAKAMURA CHEMICAL (471.3 g), each PDMSMA (78.0 g) and 2-ethylhexyl acrylate (EHA) manufactured by TOAGOSEI (693.3 g) for the polymerization reaction at 85°C for 3 hours in ethanol manufactured by KISHIDA CHEMICAL (1615.4 g). The mixture was dried at 85°C under atmospheric pressure for 2 hours after the polymerization reaction and then dried at 60°C under reduced pressure for 40 minutes to produce a concentrate. Methanol manufactured by KISHIDA CHEMICAL (6320.0 g) and water (600.0 g) were added to the concentrate (1234.8 g), and the mixture was stirred for 20 minutes. The mixture was stood still for 1.5 hours after stirring, and the supernatant was removed by decantation to obtain precipitate. Methanol (6320.0 g) was added to the precipitate, and the mixture was stirred for 20 minutes. The mixture was stood still for 1.5 hours and then the supernatant was removed by decantation. The precipitate was washed by repeating this procedure 3 times. The washed precipitate was dried at 40°C under reduced pressure for 1 hour to obtain a copolymer.

### (2) Evaluation of turbidity

A turbidity standard solution containing kaolin particles for turbidimetric test ("Turbidity Standard Solution (Turbidity: 100)" manufactured by FUJIFILM Wako Pure Chemical) was diluted with distilled water to prepare samples having turbidity of 0 to 50 by kaolin.

The absorbance at 660 nm of the turbidity samples was measured using an absorption spectrometer ("UV-1700" manufactured by Shimadzu) in accordance with JIS K0101: 2017 Testing methods for industrial water and Japanese Pharmacopoeia, Eighteenth Edition. The absorbance and the turbidity were plotted to obtain the approximate equation of y=382.38x+0.1836. The determination coefficient R² was very high as 0.9992 and it was thus indicated that the measured absorbance value bears a proportionate relationship to turbidity.

The absorbance at 660 nm of each copolymer (1 mL) produced by Example 1(1) was similarly measured, and the turbidity was determined using the standard curve. The result is shown in Table **1.**

**Table 1**

| Copolymer | Mw | Visual contact | Absorbance | Converted turbidity value |
|---|---|---|---|---|
| 1 | 1562 | slight white turbidity | 0.0280 | 11.1 |
| 2 | 1461 | white turbidity | 0.0313 | 12.4 |
| 3 | 1376 | transparent | 0.0012 | 0.8 |
| 4 | 1271 | transparent | 0.0039 | 1.8 |
| 5 | 1269 | transparent | 0.0018 | 1.0 |
| 6 | 1187 | transparent | 0.0006 | 0.5 |
| 7 | 1187 | transparent | 0.0039 | 1.8 |

It was experimentally demonstrated as the result shown in Table 1 that when the weight average molecular weight of raw material PDMSMA is large, the copolymer solution tends to have white turbidity, and at least when the weight average molecular weight of raw material PDMSMA is 1376 or less, the turbidity of the copolymer can be remarkably reduced, though there is measurement error in the low absorbance range.

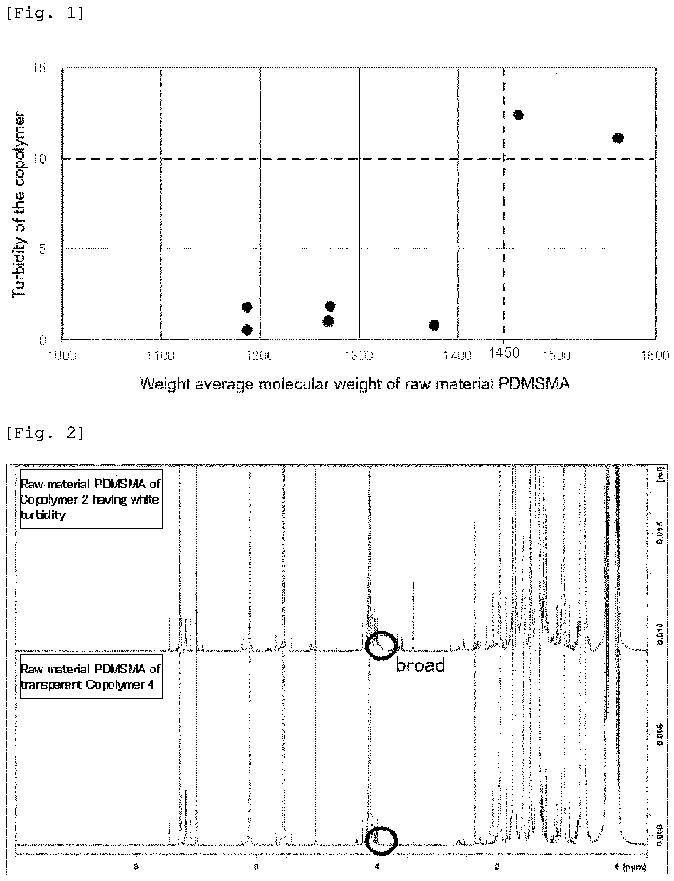

The weight average molecular weight of raw material PDMSMA and the turbidity of the copolymer were plotted. The result is shown as Figure 1. It was found that the PDMSMA having weight average molecular weight of 1450 or less has to be used for suppressing the turbidity of the copolymer to 10 or less.

### (3) Analysis

The PDMSMA used as the raw material of the copolymer 2 having white turbidity and the PDMSMA used as the raw material of the transparent copolymer 4 were analyzed by ¹H NMR. The result is shown as Figure 2.

The peak around 4 ppm in the NMR chart of the PDMSMA used as the raw material of the copolymer 2 having white turbidity is broader than that of the PDMSMA used as the raw material of the transparent copolymer 4 as the result shown in Figure 2. Since the peak is a peak of the methyl group of a dimethylsiloxane group, the PDMSMA having large weight average molecular weight may be easily polymerized together to precipitate the produced oligomer from the copolymer 2 and thereby cause white turbidity.

It was therefore indicated that the silicone monomer having weight average molecular weight of the prescribed value or less, specifically 1450 or less, has to be used for suppressing white turbidity of the copolymer.

## Claims

1. A method for producing an antithrombogenic material, comprising the step of copolymerizing an alkyl (meth)acrylate represented by the following formula (I), a silicone (meth)acrylate represented by the following formula (II), and an alkoxypolyethylene glycol (meth)acrylate represented by the following formula (III),
wherein the weight average molecular weight of the silicone (meth)acrylate is 1450 or less. 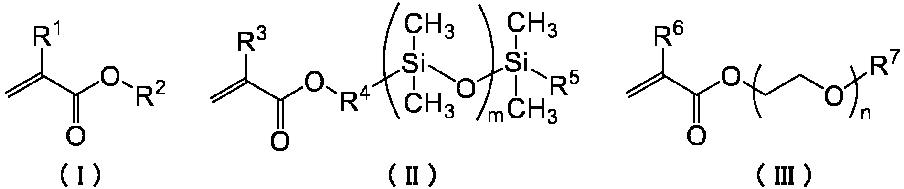
wherein
R¹ is a hydrogen atom or a methyl group,
R² is a C₆₋₂₀ alkyl group, a C₆₋₁₂ aromatic hydrocarbon group, or a C₆₋₁₂ aromatic hydrocarbon-C₁₋₆ alkyl group,
R³ is a hydrogen atom or a methyl group,
R⁴ is a C₁₋₆ alkane-diyl group,
R⁵ is a C₁₋₆ alkyl group,
R⁶ is a hydrogen atom or a methyl group,
R⁷ is a C₁₋₆ alkyl group,
m is an integer of 1 or more and 50 or less,
n is an integer of 2 or more and 10 or less.

2. The method according to claim 1, wherein the weight average molecular weight is more than 1000.

3. The method according to claim 1, wherein 1.5 times or more by mole and 2 times or less by mole of the alkyl (meth)acrylate is used to the alkoxypolyethylene glycol (meth)acrylate.

4. The method according to claim 1, wherein 0.01 times or more by mole and 0.1 times or less by mole of the silicone (meth)acrylate is used to the alkoxypolyethylene glycol (meth)acrylate.

5. The method according to claim 2, further comprising the step of concentrating a reaction mixture of the copolymerizing step under reduced pressure.

6. A method for producing a medical device,
wherein the medical device is contacted with blood,
the method comprising the steps of:
copolymerizing an alkyl (meth)acrylate represented by the following formula (I), a silicone (meth)acrylate represented by the following formula (II), and an alkoxypolyethylene glycol (meth)acrylate represented by the following formula (III) to produce a copolymer, and
applying the copolymer or a solution thereof on a surface of a medical device,
wherein the weight average molecular weight of the silicone (meth)acrylate is 1450 or less. 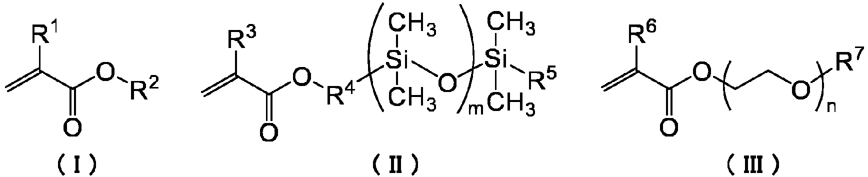 wherein
R¹ is a hydrogen atom or a methyl group,
R² is a C₆₋₂₀ alkyl group, a C₆₋₁₂ aromatic hydrocarbon group, or a C₆₋₁₂ aromatic hydrocarbon-C₁₋₆ alkyl group,
R³ is a hydrogen atom or a methyl group,
R⁴ is a C₁₋₆ alkane-diyl group,
R⁵ is a C₁₋₆ alkyl group,
R⁶ is a hydrogen atom or a methyl group,
R⁷ is a C₁₋₆ alkyl group,
m is an integer of 1 or more and 50 or less,
n is an integer of 2 or more and 10 or less.

7. An antithrombogenic material,
comprising a copolymer of an alkyl (meth)acrylate represented by the following formula (I), a silicone (meth)acrylate represented by the following formula (II), and an alkoxypolyethylene glycol (meth)acrylate represented by the following formula (III),
wherein turbidity is 10 or less. 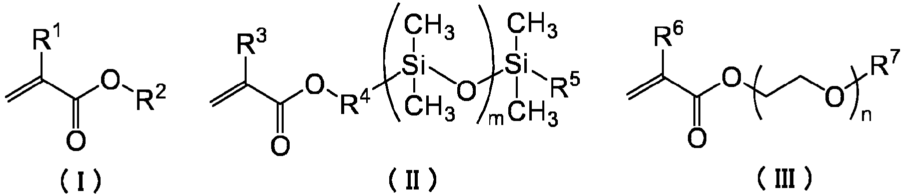 wherein
R¹ is a hydrogen atom or a methyl group,
R² is a C₆₋₂₀ alkyl group, a C₆₋₁₂ aromatic hydrocarbon group, or a C₆₋₁₂ aromatic hydrocarbon-C₁₋₆ alkyl group,
R³ is a hydrogen atom or a methyl group,
R⁴ is a C₁₋₆ alkane-diyl group,
R⁵ is a C₁₋₆ alkyl group,
R⁶ is a hydrogen atom or a methyl group,
R⁷ is a C₁₋₆ alkyl group,
m is an integer of 1 or more and 50 or less,
n is an integer of 2 or more and 10 or less.
